# EUROPEAN PATENT APPLICATION

(11) **EP 1 806 154 A1**
(43) Date of publication of application: **11.07.2007**
(21) Application number: 07100149.9
(22) Date of filing: 05.01.2007
(51) Int. Cl.: A61L 27/02, A61L 31/08

(54) **High velocity spray technique for medical implant components**

(30) Priority: 05.01.2006 US 325790
(71) Applicant: Howmedica Osteonics Corp., Mahwah, NJ 07430 (US)
(72) Inventor: Lawrynowicz, Daniel E., NEW YORK, 12518 (US); Wang, Aiguo, WAYNE, 07470 (US); Jones, Eric, LIMERICK (IE)
(74) Representative: Texier, Christian

(57) **Abstract**

Method of providing a desired material on at least a portion of a surface of a substrate of a component, such as a medical implant component. The method may comprise the steps of arranging the component in a holding fixture which is capable of holding the component at atmospheric or substantially atmospheric pressure, and spraying particles of the desired material at a predetermined high velocity toward the at least one portion of the surface of the substrate so as to enable a layer of the material to be accumulated thereon. The spraying may be performed at atmospheric or substantially atmospheric pressure. The desired material may be a reactive type material, such as titanium or an alloy thereof. The method may enable a high density coating or layer of the material to be provided without the use of a post spray thermal consolidation process.

## Description

### FIELD OF INVENTION

The present invention relates to technique for spraying a desired material onto a component and, more particularly, to such technique for spraying a desired material at a relatively high velocity onto a medical implant type component.

### BACKGROUND OF THE INVENTION

A material may be added to a component (such as a medical implant component) by a number of different techniques. One such technique is spraying. Spraying may be performed by a number of different methods including thermal spraying methods such as plasma spraying, high velocity oxygen fuel (HVOF) spraying, and so forth. Generally, in performing such thermal spraying methods, particles or powder of the material to be sprayed may be injected into a gas (such as helium, argon, nitrogen, hydrogen, or the like) and the gas and material may be projected from a spray nozzle at a relatively high velocity. Further, in thermal type spray methods, a high temperature plasma or gas flame may be utilized. Such flame temperature may be over 2000 degrees Centigrade. Additionally, during such spraying methods, particles of the material may be heated to a relatively high temperature and the component itself may be pre-heated.

Thus, during a thermal spray process, particles of a desired spray material may be sprayed at a relatively high velocity toward the surface of the medical implant component. Additionally, such particles may have a relatively high temperature.

After the spraying operation is completed, the medical implant component may be subjected to a post thermal spray consolidation or heat treatment process, such as vacuum sintering, hot isostatic pressing, and so forth. Such heat treatment or post thermal spray consolidation process may be performed to improve the density of the coated medical implant component.

As previously indicated, the above-mentioned thermal spraying methods may be utilized to add a coating of a desired material to a desired portion of a medical implant component. A material that is commonly added to or coated onto a medical implant component is titanium or a titanium alloy. Since titanium may readily react or oxidize when exposed to oxygen which may cause it to burn or result in a spontaneous combustion or formation of undesirable brittle phases, titanium may be considered a reactive type material or metal. Due to such reactive nature of titanium or an alloy thereof, thermal spraying of such material is typically performed in a closed chamber at relatively low pressure which may be close to or at a vacuum. As is to be appreciated, the cost of such chamber and of performing such spraying in a vacuum or near vacuum conditions is relatively high.

Furthermore and as previously indicated, in thermal spraying, particles of the material may be heated to a relatively high temperature and the medical implant component may be pre-heated and afterwards the component may be heat treated. Such heating, along with the impacting of the particles on the surface of the medical implant component at a high velocity, may cause an alteration of the metallurgical properties of the particles and/or the medical implant component. Such alteration may adversely affect the desired properties of the coated medical implant component. As an example, heating of the particles and/or the medical implant device during the spraying operation and/or during the heat treatment operation may cause the grain size of the particles of the coating material and/or of the substrate of the medical implant component to be significantly increased as compared to the grain size thereof prior to such heating. Such increased grain growth may adversely affect material properties of the coated medical implant component. For example, the increased grain growth may lower the fatigue properties, lower the ductility, and lower the toughness of the respective materials of the coated medical implant component.

Accordingly, it would be advantageous to provide a technique for enabling a desired material, such as a reactive type material, an alloy thereof, or a composite containing a reactive material or alloy, to be sprayed onto a surface of a medical implant component so as to provide an acceptable density value which would not result in a substantial reduction of properties such as grain size of the respective material(s) and which would be cost efficient.

### SUMMARY OF THE INVENTION

In accordance with an aspect of the present invention, a method of providing a reactive material on at least one portion of a surface of a substrate of a medical implant component is provided. Such method may comprise the steps of arranging the medical implant component in a holding fixture in which the holding fixture is capable of holding the medical implant component at atmospheric or substantially atmospheric pressure, and spraying particles of the reactive material at a predetermined high velocity toward the at least one portion of the surface of the substrate of the medical implant component so as to enable a layer of the reactive material to accumulate on the at least one portion of the surface of the medical implant component, wherein the spraying step is performed at atmospheric or substantially atmospheric pressure.

The spraying operation may be performed at atmospheric or near atmospheric conditions, that is, atmospheric pressure and temperature. As such, present spraying operation may not be performed in a vacuum or a near vacuum condition. Additionally, the predetermined high velocity may be at subsonic, sonic, and/or supersonic level(s). As an example, such high velocity may have a value of at least approximately 200 meters/second.

By use of the present method, the grain size associated with the particles may be only slightly changed due to the impact of the particles onto the surface of the medical implant component. More specifically, the grain size of the particles after impacting the outer surface of the medical implant component may be within approximately 25% or less of the grain size of the particles prior to impacting the outer surface of the substrate of the medical implant component.

Furthermore, by use of the present method, the reactive material may be provided on at least one portion of the surface of the substrate of the medical implant component with a relatively high density without the use of a post spray thermal consolidation process. Such density may have a value of approximately 99% or higher, in which the density is the percentage of the amount of reactive material per unit volume.

In accordance with another aspect of the present invention, a medical implant component is provided which may comprise a substrate formed from a first material and having an outer layer formed from a second material over at least a portion thereof. The outer layer may have a density of approximately 99% or higher, in which the density is equal to the percentage of the amount of the second material per unit volume and the outer layer has a thickness of at least approximately 25 microns.

The medical implant component may be any one of a femoral knee component, a tibial tray, a patella button, a femoral stem, a femoral head, an acetabular cup, a glenoid/humeral component, or a spinal implant.

### BRIEF DESCRIPTION OF THE DRAWINGS

A more complete appreciation of the subject matter of the present invention and the various advantages thereof can be realized by reference to the following detailed description in which reference is made to the accompanying drawings wherein like reference numbers or characters refer to similar elements.

Figure 1 is a diagram of a spray apparatus to which reference will be made in explaining an embodiment of the present invention; and

Figure 2 is a diagram of a medical implant component in accordance with an embodiment of the present invention.

### DETAILED DESCRIPTION OF THE PREFERRED EMBODIMENTS

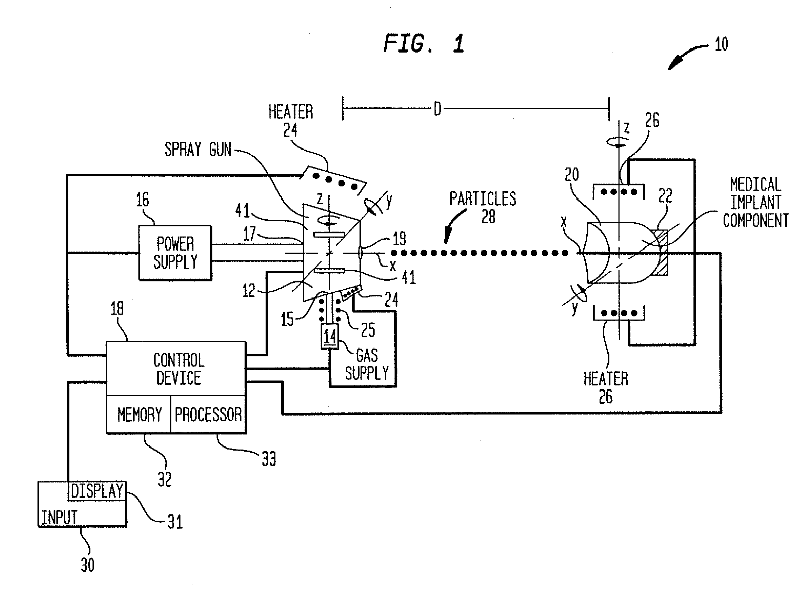

A technique for enabling a component, such as a medical implant component, to be sprayed with a desired material in accordance with an embodiment of the present invention will now be described initially with reference to system 10 illustrated in Figure 1. As shown therein, such system may generally include a spray nozzle or gun 12, a control device 18, and a holding fixture 22.

The spray gun 12 may include two inlets, a gas inlet 15 and a powder feed inlet 17. The gas inlet 15 may be adapted to receive a gas from a gas supply 14 under relatively high pressure. Such gas may be a low density gas such as helium which may enable higher gas velocities as compared to lower density gases. The powder feed inlet 17 may be adapted to receive material to be sprayed in a powder or small particle form from a powder supply 16 under relatively high pressure. The spray gun 12 may include one or more internal chambers for receiving the gas and the spray material and for directing the spray material toward an outlet 19 from which the powder or particles 28 may be supplied. Additionally, the chamber or chambers may be configured so as to accelerate the material. As a result, the powder or particles 28 may be supplied or propelled from the outlet 19 at a predetermined relatively high velocity. Such predetermined velocity may have a value in the range between approximately 200 meters/second and up to but not over sonic velocity. Alternatively, the predetermined velocity may be equal to sonic velocity and/or may be over sonic velocity so as to be at supersonic velocity. The actual predetermined velocity may be determined based on the density and/or mass of the spray material.

Instead of a single spray gun 12, the system may have two or more spray guns each operable to spray a different material. For example, one spray gun may be adapted to spray a ceramic material and another spray gun may be adapted to spray a metal material. As is to be appreciated, such arrangement may include two or more powder supplies 16, and may include two or more gas supplies 14. Alternatively, instead of two or more separate spray guns, the present system may have a single spray gun which is adapted to receive two or more different materials and to propel the same therefrom. In this arrangement, the spray gun may include separate inlets, chambers, and outlet for each material. The use of such arrangements may enable a homogeneous coating containing two or more materials to be applied to the desired surface.

Accordingly, the spray material may be merely propelled from the outlet 19 of the spray gun 12 without the use of a high temperature flame or the like and/or without being subjected to relatively high temperature. As a result, the material which is supplied or propelled from the spray gun 12 may be at a relatively low temperature. That is, unlike in a thermal type spray process wherein a high temperature flame (which may be over 2000 degrees Centigrade) may be utilized which may exit the spray gun and/or wherein the spray material may exit the spray gun at a relatively high temperature, the material which may exit the spray gun 12 of the present system 10 is at a relatively low temperature, such as less than approximately 150-200 degrees Fahrenheit. Further, the temperature of the spray material from the time which it exits the outlet 19 to just prior to impact may be less than the melting point of the spray material. Furthermore, the spray material may remain completely or substantially completely in a solid state from the time of being sprayed to the time prior to impact.

A component, such as a medical implant component 20, may be positioned or held in place by the holding fixture 22. The medical implant component 20 may be any one of a femoral knee component, a tibial tray, a patella button, a femoral stem, a femoral head, an acerabular cup, a glenoid/humeral component, or a spinal implant. As is to be appreciated, the medical implant component 20 may be generally arranged such that the surface of the medical implant component to be sprayed faces the spray gun 12. Such spray surface may be a so-called bearing surface, that is, a surface operable to engage or mate with a corresponding surface in another or mating component or with a bone, cartilage and so forth of a patient. Additionally, the holding fixture 22 may be positioned within the system 10 such that the medical implant component 20 (when held by the holding fixture) is positioned at a distance D from the spray gun 12. Such distance D may have a value of approximately 1 to 4 inches.

One or both of the spray gun 12 and the holding fixture 22 may be operable to move and/or rotate. For example, the spray gun 12 may be operable to rotate about one or both of the Y and Z axes, and/or the holding fixture 22 with the medical implant component 20 held therein may be operable to rotate about one or more of the Y and Z axes as illustrated in Figure 1. Additionally, the spray gun 12 and/or the holding fixture 22 (with the medical implant component 20 held therein) may be operable to move in a direction along any one or ones of the X-axis (i.e., toward or away from each other), the Y-axis, and/or the Z-axis.

As a result of the above-described rotation and/or movement, the stream of particles 28 may be moved relative to the component 20. Accordingly, the spray gun 12 may be able to spray particles 28 at the entire desired surface or portion of the medical implant component 20 during a spray operation.

The gas utilized in the spraying process, and/or the powder or particles 28 to be sprayed, and/or the medical implant component 20 may be heated during the spray operation. In this regard, heaters 25 may be arranged on or near the gas supply 14 or the exit thereof so as to cause the gas to be heated, heaters 24 may be arranged on or adjacent to the spray gun 12 so as to cause the powder or particles 28 to be heated, and heaters 26 may be arranged on or adjacent to the fixture 22 and/or the medical implant component 20 so as to cause such component to be heated. Additionally, the particles 28 may be electrically charged by use of a charging device 41. Such charging device 41 may be located within the spray gun 12 and may be adapted to impart an electrical charge to the particles as they pass by.

The control device 18 may include a memory 32 and a processor 33. The memory 32 may have stored therein a number of programs or algorithms usable to operate the system 10. Such programs or algorithms may be operating programs for running the system 10 and/or may include look-up tables or the like usable for generating control signals. The processor 33 may be operable to generate a control signal or signals and to supply such signal(s) to the appropriate one or ones of the devices within the system 10, as herein below more fully described.

The control device 18 may be coupled to an input 30. Such input 30 may include a keyboard type unit and may also include or may be coupled to a display 31. The input unit 31 may be operable to enable an operator to enter a desired command and/or operational information. The control device 18 may be further coupled to a number of or all of the devices in the system 10. Such connection(s) may be provided by a wire, cable, data bus, or the like coupled between the control device 18 and the device(s) of the system 10. Alternatively, such connection(s) may be provided by a wireless means.

As previously indicated, the processor 33 of the control device 18 may be operable to generate one or more control signals and to supply the same to the appropriate one or ones of the devices. More specifically, the control device 18 may be coupled to one or more of the spray gun 12, the gas supply 14, the powder supply 16, the holding fixture 22, the heater 24, the heater 25, and the heater 26; and may be operable to generate and supply control signals thereto so as to control the operation of the same. That is, in response to an input or command from an operator by way of input 30, the processor 33 of the control device 18 may generate an appropriate control signal or signals and cause the same to be supplied to the respective one or ones of the devices of the system 10. For example, in response to an input command from the operator to initiate a spray operation, the control device 18 may generate a gas supply signal and may supply the same to the gas supply 14 so as to control the supply of gas therefrom, and may generate a particle or powder supply signal and may supply the same to the powder supply 16 so as to control the supply of powder therefrom. Such control signals may control the amount of particles 28 supplied from the spray gun 12 and the velocity at which such particles are supplied therefrom. Additionally, the control device 18 may generate movement and/or rotational control signals and may supply the same to the appropriate one or ones of the spray gun 12 and/or the holding fixture 22. Such movement and/or rotational control signals may cause the spray gun 12 and/or the holding fixture 22 (with the medical implant component 20) to be moved/rotated accordingly during the spray operation. Furthermore, if requested by the operator or if appropriate, the control device 18 may generate heating control signals and may supply the same to the appropriate one or ones of the heaters 24, 25, and/or 26. Such heating control signals may cause the heaters 24, 25, and/or 26 to be activated, set to a desired temperature(s), and/or maintained thereat for a predetermined or specified time interval. As a result thereof, the particles 28 and/or the medical implant component 20 may be pre-heated to a desired temperature or temperatures. Additionally, the processor 33 may be operable to receive a feed back type signal or signals regarding the operation of any one or ones of the devices and to use the information therefrom to adjust the appropriate control signal(s). For example, the processor 33 may receive a feed back type signal indicating that the fixture 22 has been rotated too far about the Z-axis. In response thereto, the processor 33 may adjust the control signal for the fixture 22 accordingly.

The material to be sprayed may be in powder-like form or may have particles with less than a predetermined size, such as less than approximately 100µm. Such spray material may be a reactive material, that is, a material which may readily react or oxidize when exposed to oxygen. Examples of such reactive material may include titanium (Ti), zirconium (Zr), aluminium (Al), or an alloy thereof. Alternatively, the spray material may be a composite material such as a so-called cermet or ceramic metal composite type material having a reactive material or an alloy thereof. Furthermore, the spray material may be a mixture of any two or more types of materials, such as a mixture of any two or more of the materials described herein.

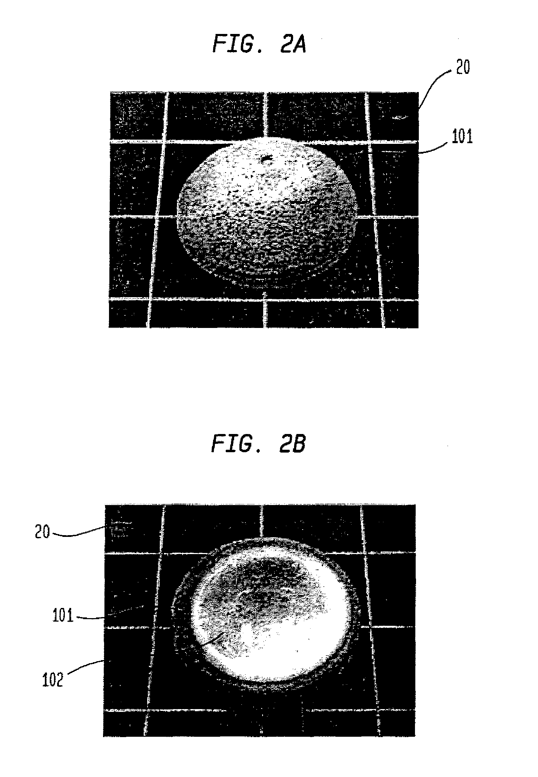

Furthermore, the material to be sprayed may be same material as that of the substrate of the medical implant component 20. That is, and with reference to Figures 2a and 2b, the medical implant component 20 may include a part or substrate portion 101 and a coating layer 102 formed on an inside surface of the substrate portion. In such situation, the material of the substrate 101 may be the same as the material used for the coating layer 102. Alternatively, the coating material may be different from the material of the substrate portion 101. For example, the substrate portion may be formed from any biocompatible metal or an alloy thereof such as cobalt chromium (CoCr) or an alloy thereof, titanium (Ti) or an alloy thereof, zirconium (Zr) or an alloy thereof, tantalum (Ta) or an alloy thereof, niobium (Nb) or an alloy thereof, or stainless steel; and the coating material may be a ceramic type material or a so-called cermet or ceramic metal composite type material. For instance, the ceramic type material may be an oxide, carbide, nitride, or nitro-carbide of any of the following elements: silicon (Si), titanium (Ti), tantalum (Ta), tungsten (W), zirconium (Zr), niobium (Nb), chromium (Cr), and aluminium (Al); and the cermet type material may be any of the previously mentioned materials and Ti and its alloys, cobalt chrome and its alloys, Zr metal and its alloys, stainless steel, and Ta and its alloys. Furthermore, materials, such as silver (Ag) or silver oxide, may be added to the metal or material for the substrate portion or to the sprayed material so as to enhance certain properties thereof such as anti-microbial properties.

Thus, the materials which may be used as the spray material and/or the material for the substrate may be a metal type material, a ceramic type material, a polymer and/or filled polymer type material, and/or a cermet material. An example of a polymer may be a polyetheretherketone (PEEK) and an example of a filled polymer type material may be a carbon fiber reinforced polyetheretherketone (PEEK). Examples of the other materials may include all biomaterials, including Ti and alloys, CoCr alloys, stainless steel alloys, Ta and alloys, Nb and alloys, zirconium and alloys (for metal type materials); alumina, zirconia, alumina-zirconia composites, silicon carbide & nitride, chrome oxide & carbides, synthetic diamond, and pyrolytic carbon (for ceramic type materials); and chrome carbide-cobalt chrome, chrome oxide-cobalt chrome, zirconium-zirconium carbide, titanium-titanium carbide, and stainless steel-titanium carbide for metal-ceramic composites or cermets. Accordingly, a metal material may be sprayed onto a ceramic or metallic substrate, and a cermet material may be sprayed onto a metallic substrate. However, the present technique is not limited to these particular combinations of materials.

Additionally, the temperature of the material may be relatively low (e.g., 150-200 degrees Fahrenheit or less) throughout most, if not all, of the spray operation. That is, as previously indicated the temperature of the material exiting the spray gun 12 may be approximately 150-200 degrees Fahrenheit or less. Except for a possible brief temperature spike that may occur at the time of impact of the material onto the surface of the substrate, the temperature of the material may not exceed the exit temperature. As a result of such low temperatures, a so-called amorphous type material may also be used. Such amorphous type material may be obtained by cooling a material from a relatively high temperature fast enough so as to prohibit or substantially prohibit the formation of a lattice of crystals. The obtained phase of the material may be maintained as long as the material is not subjected to high temperatures.

During operation, a component (such as the medical implant component 20) may be properly arranged within the fixture 22. The gas supply 14 and the powder supply 16 may be supplied with the appropriate gas and material, respectively. As an example, such gas may be helium and such material may be titanium. By use of the input 30, the operator may input commands or parameters pertaining to the spray operation. Such commands or parameters may identify the desired portion or portions of the component 20 to be sprayed, may indicate the desired thickness of the coating layer, may indicate whether or not the component, the powder, and/or the gas should be heated, and/or may indicate when the operation should commence. As a result of the inputted commands or parameters, the processor 33 may generate the appropriate control signal or signals and may cause the same to be supplied to the appropriate one or ones of the devices within the system 10. As an example, the processor 33 may generate movement and/or rotation control signals and may cause the same to be supplied to the fixture 22 and/or spray gun 12 to enable the movement and/or rotation thereof so as to permit the desired portion(s) of the component 20 to be sprayed with the spray material. Alternatively, processor 33 may select one or more program(s), algorithm(s), and/or look-up table(s) pertaining to the desired movement(s) and/or rotation(s) of the fixture 22 and/or spray gun 12 from among those previously stored in the memory 32 and may utilized the same to control the movement(s) and/or rotation(s) of the fixture and/or spray gun.

After the commands have been inputted, the spray operation may be initiated. The spray operation may cause the spray material (titanium) to be propelled at a relatively high velocity (such between approximately 200 meters/second and sonic velocity) without being subjected to high temperatures. As an example, the present spray gun may not produce a high temperature flame at the exit thereof, unlike in at least one thermal type spraying process. Even if the gas, material, and/or component are heated during the spray operation, the associated temperatures may be relatively low. As such, the present spraying process may be considered a cold temperature spray process. Furthermore, and as is to be appreciated, the present spray operation may not take place in a vacuum. Instead, such spray operation may take place in air at atmospheric pressure and/or temperature.

The spray operation may continue until the entire desired portion(s) is coated with the desired thickness of material. Such desired thickness may have a value of 25 microns or more. Afterwards, the spray operation may be terminated and the component 20 removed from the fixture 22. Since the temperatures during the spray operation are relatively low, the temperature of the component 20 after the spray operation is completed may be relatively low. For example, such component temperature may be less than approximately 150 to 200 degrees Fahrenheit. After removal, the component 20 may be machined and/or polished to the desired size or shape or surface roughness.

The present spray operation may be utilized to provide a coating layer having a desired thickness (such as 25 microns or more) on the desired surface of the substrate of the medical implant component 20. Alternatively, the present spray operation may be utilized to provide a relatively thick layer of the spray material on the desired surface of the substrate of the medical implant component 20. The thickness of such layer may be 1, 2, 3, 4, 5 millimeters or more. In fact, there may be no limit as to the thickness of such layer.

Examples of several applications for the present invention will now be described. It should be noted that these applications are provided as examples and are not intended to limit the use of the present invention to any specific situation.

For example, a metal type material (such as titanium or an alloy thereof) may be sprayed onto a substrate formed of a ceramic type material. In one situation, an acetabular ceramic insert may be sprayed with titanium or a titanium alloy on an outer surface thereof so as to form an ongrowth surface on a so-called monoblock acetabular cup. In another situation, a ceramic insert may be sprayed with titanium or a titanium alloy along with a salt (or other such material) on an outer surface thereof after which the salt may be removed so as to form a porous three dimensional ingrowth surface on a monoblock acetabular cup. In yet another situation, a ceramic insert may have its outer surface sprayed with titanium or an alloy thereof and afterwards such surface may be machined so as to be couplable to a mating modular component for attachment to a bone of a patient.

As another example, a ceramic, a cermet type material or a composite type material that has at least a predetermined amount of a metal material (such as approximately 5% or more) may be sprayed onto a surface (such surface may be an outside surface or an inner or concave type surface) of a substrate formed of a metal type material. Afterwards, such surface may be machined and/or polished so as to form a so-called bearing surface.

Additionally, prior to spraying the material onto the substrate a pre-spray process may be performed. For example, prior to spraying a metal type material (e.g., titanium or an alloy thereof) onto a substrate formed of a ceramic type material, the surface to be sprayed may be metalized. The metallization may be achieved by use of a number of processes such as ion implantation, plating, physical vapor deposition (FVD), or chemical vapor deposition (CVD).

Further, the spray material may be sprayed onto an sacrificial expendable substrate. For example, the substrate may be formed of a sacrificial material (e.g., salt, wax, sugar, a polymer which may be soluble, a metal which may be machined away or dissolved with acid, and so forth) which may be removed after the spraying process is completed so as to leave a component formed only or substantially only from the spray material.

Furthermore, the spray material may be utilized to encapsulate or isolate a material which is non-biocompatible. As is to be appreciated, such situation may be beneficial in order to encapsulate a part of a medical implant component formed from a non-biocompatible material.

Additionally, as previously indicated, the present invention may enable one or more materials to be added or sprayed onto a substrate formed of a different material. With regard to the different materials, the sprayed material or materials may have a thermal coefficient of expansion which may be different even substantially different from that of the substrate material. For example, the difference between the thermal coefficient of expansion of the coating material (TCE_{c}) and the thermal coefficient of expansion of the substrate material (TCEₘ) may be equal to or more than approximately 1.0 X 10⁻⁶ /C, (where C is degrees Centigrade).

Therefore, the present invention provides a spray technique wherein particles having a relatively small size (such as less than approximately 100µm) may be propelled at a relatively low temperature by use of a high velocity gas (such as helium) to speeds of approximately 200 meters/second or higher onto a substrate of a component, such as a medical implant component. Due to the high impact velocity of the particles onto the substrate, the particles may undergo a deformation with the substrate and form a relatively strong mechanical or metallurgical bond therewith. Depending on the material of the particles and/or substrate, such deformation may break up an oxide layer on the surface of the particles and/or the substrate, forming what may be considered an explosion weld. For example, if the particles and/or substrate are a metal material or materials, then the deformation may break up any oxide layer on the surface(s) of the particles and/or the substrate.

Due to the relatively low temperature of the spray material during the spraying operation and the relatively high velocity at which the material is propelled, the present technique enables reactive type materials (such as titanium or an alloy thereof or a composite containing such material) to be sprayed onto a medical implant component without having to be performed in a vacuum. Instead, the present technique enables such reactive type materials to be sprayed onto the medical implant component in air at atmospheric conditions, that is, at atmospheric pressure and temperature. As is to be appreciated, performing the spraying operation in air at atmospheric conditions may result in a lower operation cost as compared to performing such spraying operation in a vacuum.

Further, by use of the present method, a reactive type material may be sprayed or provided on a desired portion of the surface of the substrate of the medical implant component with a relatively high density and/or with a relatively low porosity without the use of a post spray thermal consolidation or heat treating process. Such density may have a value of approximately 99% or higher, in which the density is the percentage of the amount of reactive material per unit volume. The porosity may have a value of approximately 1% or less, in which the porosity is 100 - density. Such density and/or porosity values may be better than those obtained from other spray methods without a post spray thermal consolidation or heat treating process. For example, the porosity obtained with a HVOF or plasma spray method without a post spray thermal consolidation process may be between 3-5%. As is to be appreciated, by enabling a coating to be provided with a high density and low porosity without performing a post spray thermal consolidation process may result in a cost savings.

Furthermore, by use of the present method, the particles may not be subjected to relatively high temperatures during the actual spray operation (with the exception of a brief temperature spike which may occur upon impact as previously described). Additionally, since the present spray technique provides a coating with very acceptable density and porosity values and/or other material properties without performing a post spray thermal consolidation or heat treating process, the component with the coating layer may not be subjected to a heat treating process and, as such, may not be subjected to the prolonged or long period of heat associated therewith. As a result, the grain size associated with the particles may be only slightly changed after the impact of the particles onto the surface of the medical implant component. More specifically, the grain size of the particles after impacting the outer surface of the medical implant device may be within approximately 25% or less of the grain size of the particles prior to impacting the outer surface of the substrate of the medical implant component. Additionally, the grain may be refined and/or actually smaller in size after impact.

As a result of the small grain size change of the particles, the present spray technique may provide a coating which has improved material properties as compared to that obtained by HVOF or plasma type spray techniques with heat treating. For example, the present spray technique may result in a coating layer having higher fatigue properties, higher ductility, and/or higher toughness as compared to that obtained by HVOF or plasma spray techniques along with a post spray thermal consolidation or heat treating process.

The present spray technique may provide a number of other advantages over other techniques. For example, the present technique may reduce or eliminate thermal stress between the substrate and the sprayed layer. Also, the present technique may reduce or eliminate thermal effects on the substrate. Further, the present spray process may be faster than thermal type spray processes.

Although in the above description, helium was described as the gas used during the spray operation, the present technique is not so limited. Instead, other gases may be used during the present spray operation. For example, in addition to helium, gases such as argon, nitrogen, hydrogen, or the like may be used. Furthermore, a mixture of any combination of these gases, or a blend which contains one or more of such gases may also be used.

Additionally, although in parts of the above description the predetermined velocity at which the powder or particles 28 may be supplied or propelled from the outlet 19 was indicated to have a value in the range between approximately 200 meters/second and just less than sonic velocity, the present invention is not so limited. That is, the present invention may also be used at other predetermined velocities. For example, the powder or particles 28 may be supplied or propelled from the outlet 19 at velocities less than 200 meters/second and/or at sonic velocity and/or at supersonic velocities.

Further, although the present technique was described has being performed without any post thermal consolidation or heat treating, the present invention is not so limited. That is, if desired, a post thermal consolidation or heat treating may be performed. For example, a so-called hot isostatic pressing (HIPing) process or a so-called vacuum sintering process may be performed after the spraying operation. For more details on these and other heat treating processes and other information, see application serial number _______, filed January 5, 2006, entitled "METHOD FOR FABRICATING A MEDICAL IMPLANT COMPONENT AND SUCH COMPONENT" with inventors Daniel E. Lawrynowicz and Aiguo Wang, and application serial number __________, filed January 5, 2006, entitled "METHOD FOR FABRICATING A MEDICAL IMPLANT COMPONENT AND SUCH COMPONENT" with inventors Daniel E. Lawrynowicz, Aiguo Wang and Zongtao Zhang, both of which are hereby incorporated by reference.

Furthermore, although the present technique was described has being performed on a component such as a medical implant component, the present invention is not so limited. That is, the present spray technique may be usable on other types of medical components. For example, the present spray technique may be usable with cardiovascular components. Additionally, the present spray technique may be usable with non-medical type components.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. Method of providing a reactive material on at least one portion of a surface of a substrate of a medical implant component, said method comprising the steps of:
arranging the medical implant component in a holding fixture, said holding fixture capable of holding the medical implant component at atmospheric or substantially atmospheric pressure; and
spraying particles of the reactive material at a predetermined high velocity toward the at least one portion of the surface of the substrate of the medical implant component so as to enable a layer of the reactive material to accumulate on the at least one portion of the surface of the medical implant component,
wherein the spraying step is performed at atmospheric or substantially atmospheric pressure.

2. The method according to claim 1, wherein the spraying step is performed in air at atmospheric or substantially atmospheric conditions.

3. The method according to claim 1, wherein the predetermined high velocity has a value of at least approximately 200 meters/second but less than sonic velocity.

4. The method according to claim 1, wherein the reactive material is provided on the at least one portion of the surface of the substrate of the medical implant component with a relatively high density without the use of a post spray thermal consolidation process.

5. The method according to claim 4, wherein the reactive material provided on the at least one portion of the surface of the substrate of the medical implant component has a density value of approximately 99% or higher, in which the density is the percentage of the amount of reactive material per unit volume.

6. The method according to claim 1, wherein the particles have a grain size associated therewith, and wherein the grain size of the particles after impacting the surface of the substrate of the medical implant component is within approximately 25% of the grain size of the particles prior to impact.

7. The method according to claim 1, wherein the reactive material is a ceramic material or a ceramic metal (cermet) composite material.

8. The method according to claim 7, wherein the ceramic material is any one of an oxide, carbide, nitride, or nitro-carbide of any of the following elements: silicon (Si), titanium (Ti), tantalum (Ta), tungsten (W), zirconium (Zr), niobium (Nb), chromium (Cr), or aluminium (Al) ; and wherein the cermet composite material is formed from any (i) oxide, carbide, nitride, or nitro-carbide of any of the following elements: Si, Ti, Ta, W, Zr, Nb, Cr, or Al, and (ii) any of Ti or an alloy thereof, cobalt chrome or an alloy thereof, Zr metal or an alloy thereof, Ta or an alloy thereof, or stainless steel.

9. The method according to claim 1, wherein the substrate is formed from a biocompatible metal or an alloy thereof.

10. The method according to claim 1, wherein the medical implant component is any one of a femoral knee component, a tibial tray, a patella button, a femoral stem, a femoral head, an acetabular cup, a glenoid/humeral component, or a spinal implant.

11. Method of providing a desired material on at least one portion of a surface of a substrate of a medical implant component, said method comprising the steps of:
arranging the medical implant component in a holding fixture, said holding fixture capable of holding the medical implant component at atmospheric or substantially pressure; and
spraying particles of the desired material at a predetermined high velocity toward the at least one portion of the surface of the substrate of the medical implant component so as to enable a layer of the desired material to accumulate on the at least one portion of the surface of the substrate of the medical implant component,
wherein the spraying step is performed at atmospheric or substantially atmospheric pressure, and
wherein the particles have a grain size associated therewith, in which the grain size of the particles after impacting the surface of the substrate of the medical implant component is within approximately 25% of the grain size of the particles prior to impact.

12. The method according to claim 11, wherein the spraying step is performed in air at atmospheric or substantially atmospheric conditions.

13. The method according to claim 11, wherein the predetermined high velocity has a value of at least approximately 200 meters/second but less than sonic velocity.

14. The method according to claim 11, wherein the desired material is provided on the at least one portion of the surface of the substrate of the medical implant component with a relatively high density without the use of a post spray thermal consolidation process.

15. The method according to claim 14, wherein the desired material provided on the at least one portion of the surface of the substrate of the medical implant component has a density value of approximately 99% or higher, in which the density is the percentage of the amount of desired material per unit volume.

16. The method according to claim 11, wherein the desired material is a ceramic material or a ceramic metal (cermet) composite material.

17. The method according to claim 16, wherein the ceramic material is any one of an oxide, carbide, nitride, or nitro-carbide of any of the following elements: silicon (Si), titanium (Ti), tantalum (Ta), tungsten (W), zirconium (Zr), niobium (Nb), chromium (Cr), or aluminium (Al); and wherein the cermet composite material is formed from any (i) oxide, carbide, nitride, or nitro-carbide of any of the following elements: Si, Ti, Ta, W, Zr, Nb, Cr, or Al, and (ii) any of Ti or an alloy thereof, cobalt chrome or an alloy thereof, Zr metal or an alloy thereof, Ta or an alloy thereof, or stainless steel.

18. The method according to claim 11, wherein the substrate is formed from a biocompatible metal or an alloy thereof.

19. The method according to claim 11, wherein the medical implant component is any one of a femoral knee component, a tibial tray, a patella button, a femoral stem, a femoral head, an acetabular cup, a glenoid/humeral component, or a spinal implant.

20. Method of providing a material on at least one portion of a surface of a substrate of a medical implant component, said method comprising the steps of:
arranging the medical implant device in a holding fixture of a spray apparatus, said holding fixture capable of holding the medical implant component at atmospheric or substantially atmospheric pressure and temperature; and
spraying particles of titanium or a titanium alloy at a velocity of at least approximately 200 meters/second toward the surface of the substrate of the medical implant component so as to impact the same and enable a layer of the titanium or titanium alloy to accumulate to a desired thickness on the at least one portion of the surface of the substrate of the medical implant component, said particles having a grain size associated therewith which changes only approximately 25% or less after impacting the surface of the substrate of the medical implant component;
wherein the spraying step is performed at atmospheric or near atmospheric pressure, and
wherein said method enables the layer of titanium or titanium alloy to be provided on the at least one portion of the surface of the substrate of the medical implant component with a density value of approximately 99% or higher, in which the density is the percentage of the amount of reactive material per unit volume, without performing a post spray thermal consolidation process.

21. The method according to claim 20, wherein the medical implant component is any one of a femoral knee component, a tibial tray, a patella button, a femoral stem, a femoral head, an acetabular cup, a glenoid/humeral component, or a spinal implant.

22. A medical implant component comprising a substrate formed from a first material and having an outer layer formed from a second material over at least a portion thereof, said outer layer having a density of approximately 99% or higher in which the density is equal to the percentage of the amount of the second material per unit volume, and said outer layer having a thickness of at least approximately 25 microns.

23. The medical implant component according to claim 22, wherein the first material is the same as the second material.

24. The medical implant component according to claim 22, wherein the first material is different from the second material.

25. The medical implant component according to claim 24, wherein the first material is a biocompatible metal or an alloy thereof.

26. The medical implant component according to claim 25, wherein the second material is a ceramic material or a ceramic metal (cermet) composite material.

27. The medical implant component according to claim 26, wherein the ceramic material is any one of an oxide, carbide, nitride, or nitro-carbide of any of the following elements: silicon (Si), titanium (Ti), tantalum (Ta), tungsten (W), zirconium (Zr), niobium (Nb), chromium (Cr), or aluminium (Al); and wherein the cermet composite material is formed from any (i) oxide, carbide, nitride, or nitro-carbide of any of the following elements: Si, Ti, Ta, W, Zr, Nb, Cr, or Al, and (ii) any of Ti or an alloy thereof, cobalt chrome or an alloy thereof, Zr metal or an alloy thereof, Ta or an alloy thereof, or stainless steel.

28. The medical implant component according to claim 22,
wherein the medical implant component is any one of a femoral knee component, a tibial tray, a patella button, a femoral stem, a femoral head, an acetabular cup, a glenoid/humeral component, or a spinal implant.

29. The method according to claim 1, wherein the predetermined high velocity has a value of approximately sonic velocity.

30. The method according to claim 1, wherein the predetermined high velocity is a supersonic velocity.

31. The method according to claim 1, wherein the substrate is formed from a ceramic material.

32. The method according to claim 11, wherein the predetermined high velocity has a value of approximately sonic velocity.

33. The method according to claim 11, wherein the predetermined high velocity is a supersonic velocity.

34. The method according to claim 11, wherein the substrate is formed from a ceramic material.

35. The method according to claim 20, wherein the substrate is formed from a ceramic material.
